# EUROPEAN PATENT SPECIFICATION

(11) **EP 1 112 505 B1**
(45) Date of publication and mention of the grant of the patent: **12.07.2006**
(21) Application number: 99943154.7
(22) Date of filing: 07.09.1999
(51) Int. Cl.: G01R 31/08

(54) **LOCATING UNDERGROUND POWER CABLE FAULTS**
ORTUNG VON FEHLERN AN UNTERIRDISCHEN VERSORGUNGSKABELN
LOCALISATION DE DEFAUTS DE CABLES ELECTRIQUES SOUTERRAINS

(30) Priority: 08.09.1998 GB 9819586
(43) Date of publication of application: 04.07.2001
(73) Proprietor: EA TECHNOLOGY LIMITED, Capenhurst, Chester CH1 6ES (GB)
(72) Inventor: MILLER, Dawn, Elizabeth, Chester Cheshire CH2 3JH (GB)
(74) Representative: Cross, Rupert Edward Blount
(86) International application number: PCT/GB1999/002964
(87) International publication number: WO 2000/014557

(56) References cited:
- DE-A- 3 931 340
- DE-A- 4 001 959
- DE-A- 4 418 774
- US-A- 4 369 647
- PATENT ABSTRACTS OF JAPAN vol. 15, no. 210 (P-1208), 29 May 1991 (1991-05-29) & JP 03 057978 A (FUJIKURA), 13 March 1991 (1991-03-13)

## Description

The present invention relates to electrical faults in underground power cables, and to apparatus and methods for locating such faults.

Low voltage underground power cables fail for a variety of reasons, including deterioration of the insulation, failure of environmental protection and mechanical damage to the cable. Locating the exact point of fault can be a lengthy exercise and generally takes longer than the repair itself, particularly if a "hit-and-miss" excavation approach has to be adopted. There is also the problem of intermittent tracking faults which temporarily clear themselves as they trip a circuit protection device. When the supply is restored the fault appears to have gone, only to reappear at a later date. Such faults are notoriously difficult to locate, causing considerable disruption to supply, and consequently are very unpopular with both electricity companies and customers.

Various methods of locating faults are used, with varying degrees of effectiveness. A resistance bridge can be used to locate contact faults. The measurement is made by balancing two internal resistance arms against the two external resistance arms represented by the lengths of cable conductor either side of the fault. At balance the ratio of the internal resistance arms is equal to the ratio of the external resistance arms. By assuming that the cable has a constant resistance per unit length, the location of the fault can be derived.

In time domain reflectometry a pulse is launched into the cable from one end and will be completely or partially reflected by any fault in the cable. The velocity of propagation of the pulse through a cable depends on the cable dielectric, and can be found from literature, but these values are only approximate due to differences in type, temperature and ageing of the insulation. It is usually possible, however, to use a propagation velocity obtained from a known length of the same cable.

The velocity of pulse propagation is taken to be constant for a given cable, therefore the time taken for the pulse to return to the source is a measure of the distance to the fault. Other cable features as well as faults and ends can give rise to reflections; these include joints, changes in cable cross-section and core splits which produce minor mismatches, and tee joints and ends of tee branches which produce more significant mismatches.

Low voltage cables consist of various types of cable, earthed at many points, with solid tee joints providing services to customer's premises. Due to te complexity of low voltage networks, discrete features, even those from short circuits and breaks, cannot normally be recognised among the multitude of features making up a typical pulse echo trace. A gross mismatch will only be clearly visible if it is very close to the point at which the pulse echo equipment is connected.

In underground power cable fault detection the vast majority of pin-pointing tests are carried out using a surge generator to create noise and vibration at the fault site. The surge generator works by charging up a capacitor, the capacitance of which can be adjusted to vary the energy stored. When the capacitor is discharged into the cable a very high energy, steep fronted surge wave is launched.

If the surge has high enough voltage and capacitance to cause a breakdown it enhances energy dissipation in the fault and creates noise and vibration. In the air this creates a very loud 'bang', but with the cable buried at a depth of between a half and one metre the sound is muffled and produces a 'thump' which can sometimes be heard unassisted overground but more often can only be detected using a sensitive ground microphone.

The process is repeated every few seconds and the operator can walk over the suspect zone or along the length of cable to pinpoint the fault location.

Another method of fault detection involves checking at points along the length of the cable for the presence and direction of the electromagnetic signal created by the current produced by a steep fronted voltage impulse when surging. The equipment comprises a pick-up coil feeding a receiver with a polarisation indicator which gives the magnitude and direction of the signal. The pick-up coil is marked so that the same end is always pointed to the source to make sure that directional information is correct. The method is limited to use on ducted systems since the pick-up coil needs to be placed directly on the cable itself. This electromagnetic method is equally effective whether the fault is arcing or is a short-circuit as current flows in both cases.

US 4,369,647 discloses a gas leakage detector comprising a solid state gas sensor and a suction pump to draw gas through a gas intake path to the gas sensor. JP 03057978 discloses a gas sensor located within a CV cable connecting element. Gases generated on decomposition of a polyethylene sheath due to electric discharge diffuse along a gap in the cable sheath to the sensor.

The invention provides an electrical fault locater as set out in claim 1 and a method of locating electrical faults as set out in claim 13.

Conveniently, a number of solid state gas sensors with different sensitivity characteristics to different gases may be used simultaneously. The pattern of responses from the different sensors then provides a 'fingerprint' that may be used to identify samples of gases characteristic of underground power cable faults.

Conveniently, the solid state gas sensors may be metal oxide semiconductor gas sensors. Semiconductor gas sensors with sensing elements made of tin dioxide and with a wide range of different sensitivity characteristics to different gases are commercially available. Typically, metal oxide semiconductor gas sensors have a low conductivity in clean air. In the presence of a detectable gas the sensor conductivity increases, the conductivity response depending on the type of gas and its concentration in the air. Other types of solid state gas sensors may also be used, such as quartz crystal microbalances and surface acoustic wave devices. The response of the solid state gas sensors to detectable gases may therefore be measured other than as changes in sensor conductivity, for example as changes in resonant frequency.

Commercially available metal oxide semiconductor gas sensors typically incorporate heating elements. These heating elements may be made of platinum wire, the resistance of which changes with temperature so that the element temperature can be easily controlled by electronic circuitry. The semiconductor sensor element is typically in close proximity to the heating element, for example on the surface of a tube in which the heating element is positioned, so that the temperature of the sensing element is close to that of the heating element. Since the sensitivity characteristics of metal oxide semiconductor gas sensors change with operating temperature, the heating elements may conveniently be used to individually operate the gas sensors at temperatures suitable to optimise the response of the apparatus to particular gases and combinations of gases. Preferably therefore, the apparatus includes means to energise and control the temperatures of the heating elements and thus to control the operating temperatures of the sensors.

Preferably, at least four different solid state gas sensors may be used in the sensing chamber. Fewer sensors may compromise the ability of the apparatus to distinguish between gases characteristic of underground power cable faults and other gas combinations. However, too many different sensors will make the apparatus more bulky, complex and expensive.

Preferably, the apparatus includes a mass flow controller to control the rate of flow of a gas sample into the sensing chamber.

Preferably, the apparatus includes calibration means adapted to monitor said signals when clean air is drawn into the sensing chamber and to provide a calibration reference for use in analysing said signals. Thus the responses of the gas sensors when exposed to a clean air sample can provide reference responses from which to measure response changes when the gas sensors are exposed to a gas sample.

Metal oxide semiconductor gas sensors are sensitive to the relative humidity and temperature of the gas sample. Preferably, therefore, the apparatus may include sensors to monitor the relative humidity of the gas sample in the sensing chamber and the air temperature. Signals from these sensors may then be used in the analysis of the signals from the solid state gas sensors to compensate for variations in gas sensor response due to changes in relative humidity and air temperature. It may be only necessary or convenient to compensate for one of these two parameters. Of course, the requirement to compensate for either air temperature or relative humidity could be removed if sensors were used that had very low sensitivities to these parameters, or if the relative humidity or temperature of the gas sample were controlled by some means. The air temperature may be measured inside or outside the sensing chamber, thus corresponding to the temperature of the gas sample close to the point of measurement or the ambient air temperature.

In one embodiment of the invention, the analysis means for analysing the signals representing the responses of the solid state gas sensors are arranged to use a weighted sum of the sensor responses to identify the presence of gases characteristic of underground power cable faults. In an alternative embodiment, the analysis means are arranged to use a neural network that is trained to identify characteristic gases. In other embodiments, principle component analysis or discriminate function analysis are used to identify characteristic gases.

In one embodiment of the method, the gas is sampled from a hole in the ground surface near to the suspected cable fault and the sample gas is drawn into a sensing chamber containing at least one solid state gas sensor. Electronic circuitry connected to the gas sensors is used to produce signals representing the responses of the gas sensors. The signals are then analysed to identify the presence in the sample gas of gases characteristic of underground power cable faults.

Preferably, the sample gas is drawn into the sensing chamber using a probe which is inserted into the hole in the ground.

Conveniently, a number of holes may be drilled in the surface of the ground in a line above an underground cable. By analysing gas samples from each of the holes, the hole closest to the cable fault is indicated by determining the gas sample with the highest concentration of characteristic gases.

Embodiments of the present invention will now be described with reference to the drawings, of which:
Figure 1 is a diagram of a device of the present invention, in use with the probe drawing air from a hole in the ground in the vicinity of an underground power cable fault;
Figure 2 is a cross sectional view through the probe and sensor housing of the device of figure 1;
Figure 3 is a schematic diagram showing the principle components in the control unit of the device of figure 1; and
Figure 4 is a schematic diagram showing the electronic circuitry of the data acquisition-interface in the control unit of the device shown in figure 1.

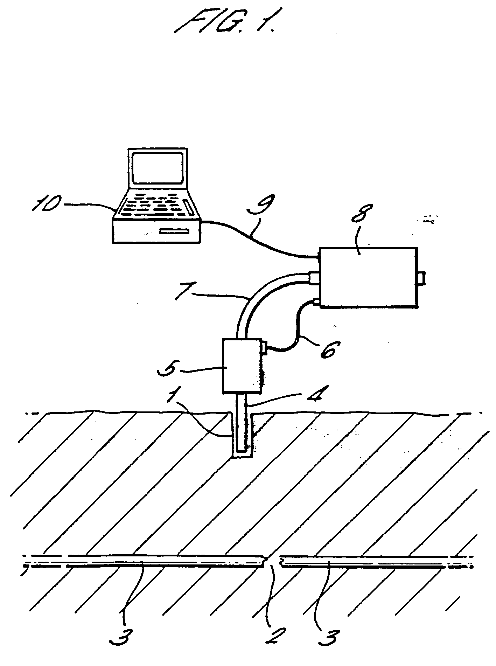

An embodiment of the present invention is illustrated in figure 1. The embodiment is shown being used to sample the air from a hole 1 in the ground in the vicinity of a fault 2 in an underground power cable 3. Air is drawn by a pump in the control unit 8 from the hole in the ground 1 through a hollow probe 4 and into a sensor unit 5 that contains an array of metal oxide semiconductor gas sensors. The sensor unit is connected to the pump in the control unit 8 by an air line 7. The array of gas sensor elements is connected to the control unit 8 by an electrical cable 6 that is used to read information from the gas sensor array.

The control unit contains an air pump and mass flow controller to draw air through the sensor unit, electronics to read and digitise the conductivities of the gas sensors and the temperature and relative humidity in the gas sensor unit, and circuitry to drive the heater elements in the gas sensors. The control unit 8 is operated by a personal computer 10 which is connected by a serial port cable. The computer calibrates the measured conductivities to account for the ambient air temperature and relative humidity and uses the results to determine a measure of the concentration in the sample air of gases typically produced by a cable fault.

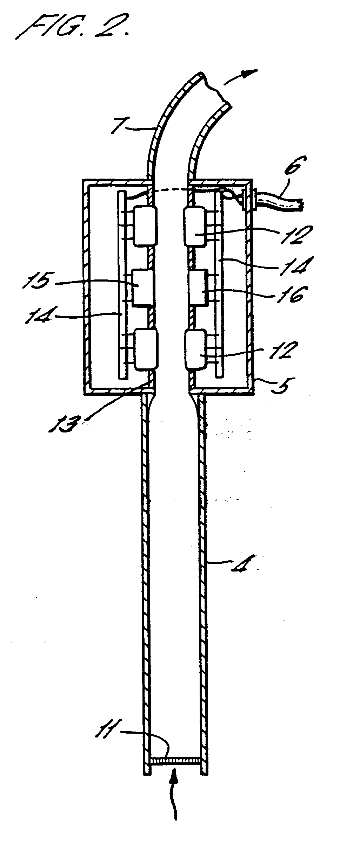

The sensor unit is illustrated in more detail in figure 2. The probe is about 10cm long and 1cm in diameter and is made of a suitably robust glastic such as PTFE. A filter 11 is fitted at the end of the probe to prevent fine particles from being drawn into the equipment in the airstream. Clean air may be drawn through the probe from time to time for calibration purposes by removing the probe from the hole in the ground. Air drawn through the probe enters a chamber 13 in the sensor unit in which several metal oxide semiconductor gas sensors 12 are mounted in such a way that their leads pass through the chamber to be mounted on printed circuit boards 14. Between four and six sensors 12 may be used to obtain an instrument that is compact, has reasonable sensitivity to gases associated with cable faults, and does not respond unduly to gases encountered in situations other than near to a cable fault. A relative humidity sensor 15 and an air temperature sensor 16 are also mounted on the printed circuit boards 14 in such a way that they respond to the air in the sensor chamber. In another embodiment the air temperature sensor is located outside the sensing chamber and measures the ambient air temperature.

The printed circuit boards 14 are connected by a cable 6 to the control unit. Air is drawn at a rate of between about 100ml and 200ml per minute through the probe and over the gas sensors by means of an air line 7 connected to the control unit. In another embodiment, the sensor chamber 13 is flushed with sample air which is then left stationary in the sensing chamber for a sampling period.

The gas sensors 12 in the sensor unit 15 are chosen to be sensitive to gases characteristic of underground power cable faults, such as decene, toluene, naphthalene and furancarboxaldehyde. Which gases are characteristic of underground power cable faults may be determined by laboratory experiments in which power cable insulation is burned or subjected to arcing activity. Alternatively, soil samples from sites of cable faults may be analysed. Mass spectrometry and gas chromatography may be used to determine the gases produced in these scenarios. Preferably, sensors should be used that exhibit a strong response to such gases but a weak response to other gases that are likely to be encountered even when there is no cable fault nearby. In the present embodiment, the sensitivity of the metal oxide semiconductor sensors to the target gases may be maximised by operating at appropriate sensor temperatures as determined by experiment or by the manufacturer's literature.

Six metal oxide semiconductor gas sensors 12 which may be suitable for use in the sensor chamber 5 of the present embodiment are made by Figaro and have the following manufacturer's codes: TGS 813, TGS 822, TGS 880, TGS 842, TGS 825, TGS 826.

While the present embodiment has been described as using metal oxide semiconductor gas sensors 12, it will be appreciated that other suitable solid state gas sensing devices may be similarly used. For example, quartz crystal microbalances incorporate a piezoelectric quartz crystal onto one face of which a gas sensitive layer is sublimed. The crystal is employed as the active element in a resonant circuit. As the mass of a detectable gas adsorbed onto the coated surface increases, the oscillation frequency decreases linearly. An uncoated quartz crystal can be used as a frequency reference to compensate for temperature fluctuations. Surface acoustic wave (SAW) devices may also be used as suitable solid state gas sensors. As gases are adsorbed onto the sensor surface of the crystal of an SAW device, the acoustic wave velocity on the surface of the crystal changes. The response of such devices to the presence of particular gases will not necessarily be in terms of conductivity, so that the circuitry used to drive and read the response of the gas sensors may be different to that described for the embodiment of the invention in which metal oxide semiconductor devices are used.

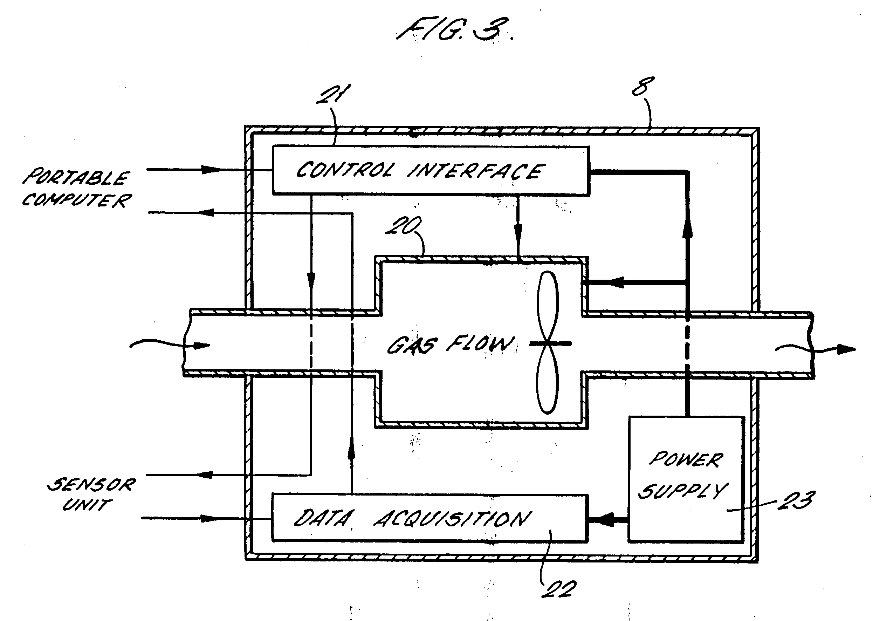

The functional parts incorporated into the control unit are illustrated in figure 3. The flow of data and instructions is shown by arrows on the lines connecting the components. Power supply is shown by heavy lines. The gas flow unit 20 incorporates an air pump and a mass flow controller to draw air through the instrument at a controlled rate. The control interface 21 contains two functional parts. These are power supply circuits for the heating elements of the gas sensors and control circuits for the gas flow unit. The control interface is itself controlled by the portable computer. The data acquisition interface 22 reads the conductivities of the gas sensors in the sensor unit. The relative humidity and air temperature sensors in the sensor unit are also read by this circuitry. This information is digitised and communicated by a serial link to the portable computer. A power supply unit 23 such as a battery is provided in the control unit to power the other components. Alternatively, an external power supply unit may be provided.

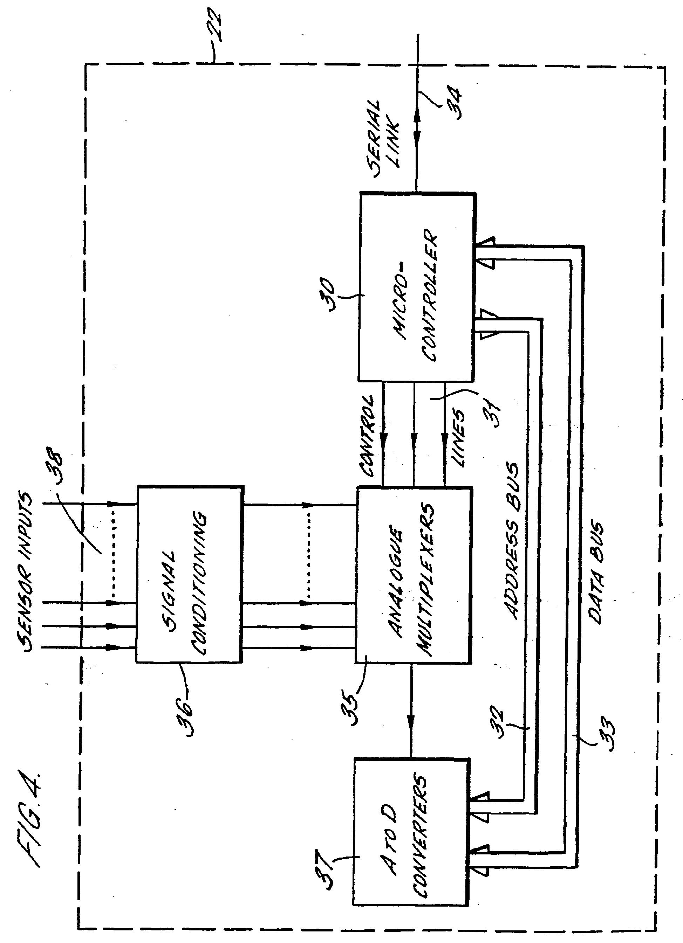

A block diagram of the data acquisition interface 22 of the present embodiment is shown in figure 4. The physical realisation of this system consists of two equally sized printed circuit boards. One board features a standard Intel 8031 micro controller subsystem 30. This incorporates program and data memory. Connections to the micro controller include control lines 31, an address bus 32, data bus 33 and a serial data link 34. The second printed circuit board hosts the analogue electronics. This includes a multiplexer sub-system 35 with 'on' resistance nulling, a signal conditioning subsystem 36 and an analogue to digital (AD) converter 37. The signal conditioning circuits 36 are connected directly to the metal oxide semiconductor gas sensors and produce voltages proportional to the resistances of the sensor elements. These signals are presented in turn to the AD converter 37 by the analogue multiplexing circuits 35.

The micro controller subsystem 30 controls the sequential selection of each sensor input 38 and also generates the control signals required by the AD converter. Each digitised voltage reading is stored in random access memory in the micro controller 30. The micro controller transmits stored sensor data to the personal computer via the serial data link 34. Serial data can also be received by the interface to allow external control of interface functions.

A portable computer may be used to provide overall control of the equipment and to interpret the sensor readings to determine a measure of the concentration of target gases characteristic of underground cable faults. Ideally, a single measure of target gas concentration is derived so that operation of the instrument in location of cable faults involves simply looking for the location where the single measure of target gas concentration is highest. Such a measure may be derived from the gas sensor responses by a variety of means. A simple weighted sum of the responses may be sufficient, where the optimum weightings for the sensor responses may be determined by calibration using soil samples from cable fault sites. Two or more sets of weightings may be desirable to correspond to two or more different cable insulation types, such as PVC and oil impregnated paper which release different characteristic gases on burning.

A more complex measure of target gas concentration may be used. For example, a neural network may be trained to discriminate between samples of the target gases and blank samples or samples containing other commonly encountered mixtures of gases. Other methods to determine target gas concentration may include principal component analysis and discriminate function analysis, or any other suitable statistical method.

The use of a number of gas sensors with different response characteristics to different gases in the sensing unit enables the apparatus to determine a multidimensional vector or fingerprint of responses against which to match responses characteristic of target gas combinations using any suitable method such as the weighted sum or neural network methods mentioned above. Thus by using a larger number of solid state gas sensors the apparatus is better able to distinguish between gas combinations characteristic of cable faults and other gas combinations that may be encountered. Too many gas sensors would make the apparatus more bulky, complex and expensive and may not significantly improve performance. Increasing the number of gas sensors would also increase the power consumption of the device and the complexity or the software and electronics. Conveniently, between four and six different gas sensors may be used, although satisfactory operation may be achieved by the careful selection of fewer sensors.

The computer receives digitised information from the temperature and relative humidity sensors in the sensor unit. These readings are used to correct the measured sensor responses. The conductivities of metal oxide semiconductor gas sensors depend strongly on these environmental factors. From time to time during operation a sample of clean air may be taken by removing the probe from the vicinity of a cable fault. The responses of the gas sensors in clean air may then provide a calibration baseline. This calibration procedure allows compensation to be made for any long term drift of the sensor responses. Alternatively, a clean air intake may be provided into the gas sensor chamber to be operated manually or automatically.

In the embodiment described above, a separate portable computer is used for instrument control, data processing and display. In other embodiments a microprocessor unit, or other suitable electronics may be included in the control unit itself. Since only a simple measure of target gas concentration is required for cable fault location, a simple alphanumeric display or row of light emitting diodes may provide sufficient data readout for satisfactory operation of the instrument. Such an embodiment may be constructed to be more portable, compact and more straightforward to operate than if a separate portable computer is used.

Underground low voltage cable faults may be located using the cable fault detection apparatus described above. The probe used to draw sample gas into the sensing chamber may be inserted in a small hole drilled in the ground in the suspected vicinity of a cable fault. The detection of significant amounts of gases characteristic of cable faults may be taken as an indication that a cable fault is indeed located nearby.

Boreholes may be made at regular intervals in the ground along the length of the buried cable and the probe of the cable fault detection apparatus inserted into each hole to sample the gases present in the soil. Where another fault location exercise has already been carried out using another means of fault detection, and an estimate of the fault position already exists, bore holes can be made short distances apart in the estimated region of the fault. The borehole position which gives the highest gas concentration measurement indicated the position of the fault.

Intermittent cable faults may be more easily found using an embodiment of the present invention. An intermittent fault may cause characteristic gases to permeate into the ground around the cable fault. During periods when the cable is functioning normally the characteristic gases may still be detected.

## Claims

1. An electrical fault locator for locating electrical faults (2) in underground power cables (3) comprising:
a plurality of solid state gas sensors (12) mounted in a sensing chamber (13), a hollow probe (4) connected to the sensing chamber and adapted for sampling gas from a hole (1) in the ground surface above a suspected cable fault (2), means to draw sample gas through the probe into the sensing chamber, electronic circuitry (22) connected to the gas sensors to produce signals representing the responses of the sensors, means (10) to analyse said signals to identify the presence in said sample gas of gases characteristic of underground power cable faults, and means (10) to indicate the result of the analysis.

2. The apparatus as claimed in claim 1 in which said solid state gas sensors (12) are solid state gas sensors with different sensitivity characteristics to different gases.

3. The apparatus as claimed in claim 1 in which the solid state gas sensors (12) are metal oxide semiconductor gas sensors.

4. The apparatus as claimed in claim 3 in which said metal oxide semiconductor gas sensors (12) incorporate heating elements, and the apparatus includes means (21) to energise and control the temperatures of said heating elements and to thus control the operating temperatures of said metal oxide semiconductor gas sensors.

5. The apparatus as claimed in any preceding claim in which there are at least four of said solid state gas sensors (12).

6. The apparatus as claimed in any preceding claim including a mass flow controller (20) to control the flow of said sample gas into said sensing chamber (13) .

7. The apparatus as claimed in any preceding claim including calibration means adapted to monitor said signals when clean air is drawn into said sensing chamber (13) and to provide a calibration reference for use in analysing said signals.

8. The apparatus as claimed in any preceding claim including a relative humidity sensor (15) positioned in said sensing chamber (13) and means connected to said relative humidity sensor to compensate the responses of the solid state gas sensors (12) for changes in relative humidity.

9. The apparatus as claimed in any preceding claim including an air temperature sensor (16) and means connected to said air temperature sensor to compensate the responses of the solid state gas sensors (12) for changes in air temperature.

10. The apparatus as claimed in any of claims 1 to 9 in which the analysis means (10) are arranged to use a weighted sum of the responses of said solid state gas sensors (12).

11. The apparatus as claimed in any of claims 1 to 9 in which the analysis means (10) is arranged to use a neural network trained to identify samples of said gases from the responses of said solid state gas sensors (12).

12. The apparatus as claimed in any of claims 1 to 9 in which the analysis means (10) is arranged to use one of the methods in the list of principal component analysis and discriminate function analysis.

13. A method of locating electrical faults (2) in underground cables (3) comprising the steps of:
drawing gas samples from locations near to a suspected electrical fault (2) of an underground cable;
exposing at least one solid state gas sensor (12) to said gas samples; and
monitoring the consequent changes in the responses of the solid state gas sensors and
determining therefrom an indication of the location of the electrical fault.

14. The method as claimed in claim 13 in which the first step comprises extracting said gas samples from holes (1) in the ground surface near to said suspected cable fault (2) and drawing said gas samples into a sensing chamber (13) containing said at least one solid state gas sensor (12), the third step of monitoring comprises using electronic circuitry (22) connected to said solid state gas sensors to produce signals representing the responses of said solid state gas sensors, and the fourth step of determining comprises analysing said signals to identify the presence in said gas samples of gases characteristic of underground power cable faults.

15. The method of claim 14 in which said gas samples are drawn into said sensing chamber (13) using a probe (4) which is inserted into said holes (1) in the ground surface.

16. The method of either claim 14 or claim 15 in which a number of said holes (1) are drilled in the ground surface in a line above an underground cable (3), said gas samples are taken from each of the holes and, and in which the highest concentration of gases characteristic of an underground power cable fault is taken to indicate the hole closest to the cable fault (2) .

17. The method of any of claims 13 to 16 in which said solid state gas sensors (12) are solid state gas sensors with different sensitivity characteristics to different gases.

18. The method of any of claims 13 to 17 in which the solid state gas sensors (12) are metal oxide semiconductor gas sensors.

19. The method of claim 18 in which said metal oxide semiconductor gas sensors (12) are operated at desired temperatures by means of heating elements incorporated therein.

20. The method of any of claims 13 to 19 in which at least four solid state gas sensors (12) are used.

21. The method of any of claims 14 to 20 in which a mass flow controller (20) is used to control the flow of said gas samples into said sensing chamber (13).

22. The method of any of claims 14 to 21 in which calibration means adapted to monitor said signals when clean air is drawn into the sensing chamber (13) are used to provide a calibration reference for use in analysing said signals.

23. The method of any of claims 14 to 22 in which the signal from a relative humidity sensor (15) positioned in said sensing chamber is used to compensate the responses of the solid state gas sensors (12) for changes in relative humidity.

24. The method of any of claims 14 to 23 in which the signal from an air temperature sensor (16) is used to compensate the responses of the solid state gas sensors (12) for changes in air temperature.

25. The method as claimed in any of claims 13 to 24 in which the step of determining uses a weighted sum of the responses of said solid state gas sensors (12).

26. The method as claimed any of claims 13 to 24 in which the step of determining uses a neural network trained to identify samples of said gases from the responses of said solid state gas sensors (12).

27. The method as claimed in any of claims 13 to 24 in which the step of determining uses one of the list of principal component analysis and discriminate function analysis.

## Patentansprüche

1. Elektrisches Fehlersuchgerät zum Orten von elektrischen Fehlern (2) in unterirdischen Starkstromkabeln (3), wobei das Gerät
- eine Vielzahl von Festkörpergassensoren (12), die in einer Messkammer (13) angebracht sind,
- eine hohle Sonde (4), die mit der Messkammer verbunden und für die Probenahme von Gas aus einem Loch (1) in der Bodenfläche über einem vermuteten Kabelfehler (2) ausgelegt ist,
- Einrichtungen zum Ansaugen von Probegas durch die Sonde in die Messkammer,
- eine elektronische Schaltung (22), die mit den Gassensoren verbunden ist, um Signale zu erzeugen, die die Reaktionen der Sensoren darstellen,
- Einrichtungen (10) zum Analysieren der Signale, um das Vorhandensein von Gasen in dem Probegas zu identifizieren, die für Fehler an unterirdischen Starkstromkabeln charakteristisch sind, und
- Einrichtungen (10) zum Anzeigen des Analyseergebnisses aufweist.

2. Vorrichtung nach Anspruch 1, bei welcher die Festkörpergassensoren (12) Festkörpergassensoren mit unterschiedlichen Empfindlichkeitseigenschaften für verschiedene Gase sind.

3. Vorrichtung nach Anspruch 1, bei welcher die Festkörpergassensoren (12) Metalloxidhalbleiter-Gassensoren sind.

4. Vorrichtung nach Anspruch 3, bei welcher die Metalloxidhalbleiter-Gassensoren (12) Heizelemente einschließen und die Vorrichtung Einrichtungen (21) zum Anregen und Steuern der Temperaturen der Heizelemente und somit zum Steuern der Betriebstemperaturen der Metalloxidhalbleiter-Gassensoren aufweist.

5. Vorrichtung nach einem vorhergehenden Anspruch, bei welcher wenigstens vier Festkörpergassensoren (12) vorhanden sind.

6. Vorrichtung nach einem vorhergehenden Anspruch mit einer Massendurchsatzsteuerung (20) zum Steuern des Durchsatzes des Probegases in die Messkammer (13).

7. Vorrichtung nach einem vorhergehenden Anspruch mit Eicheinrichtungen zum Überwachen der Signale, wenn reine Luft in die Messkammer (13) gesaugt wird, und zur Bereitstellung einer Eichbezugsgröße zur Verwendung beim Analysieren der Signale.

8. Vorrichtung nach einem vorhergehenden Anspruch mit einem Sensor (15) für relative Feuchte, der in der Messkammer (13) angeordnet ist, und mit Einrichtungen, die mit dem Sensor für die relative Feuchte verbunden sind, um die Reaktionen der Festkörpergassensoren (12) auf Änderungen der relativen Feuchte auszugleichen.

9. Vorrichtung nach einem vorhergehenden Anspruch mit einem Lufttemperatursensor (16) und mit Einrichtungen, die mit dem Lufttemperatursensor verbunden sind, um die Reaktionen der Festkörpergassensoren (12) auf Änderungen der Lufttemperatur auszugleichen.

10. Vorrichtung nach einem der Ansprüche 1 bis 9, bei welchem die Analyseeinrichtungen (10) so angeordnet sind, dass sie eine gewichtete Summe der Reaktionen der Festkörpergassensoren (12) verwenden.

11. Vorrichtung nach einem der Ansprüche 1 bis 9, bei welcher die Analyseeinrichtungen (10) so angeordnet sind, dass sie ein neurales Netzwerk verwenden, das zur Identifizierung von Proben der Gase aus den Reaktionen der Festkörpergassensoren (12) ausgebildet ist.

12. Vorrichtung nach einem der Ansprüche 1 bis 9, bei welchem die Analyseeinrichtungen (10) so angeordnet sind, dass sie eines der Verfahren in der Liste einer Hauptkomponentenanalyse und einer Unterscheidungsfunktionsanalyse verwenden.

13. Verfahren zum Suchen von elektrischen Fehlern (2) in unterirdischen Kabeln (3), wobei das Verfahren die Schritte aufweist
- Ansaugen von Gasproben von Stellen in der Nähe eines vermuteten elektrischen Fehlers (2) an einem unterirdischen Kabel,
- wenigstens einen Festkörpergassensor (12) den Gasproben Aussetzen,
- Überwachen der daraus folgenden Änderungen in den Reaktionen der Festkörpergassensoren und
- daraus Bestimmen einer Anzeige des Orts des elektrischen Fehlers.

14. Verfahren nach Anspruch 13, bei welchem
- der erste Schritt das Extrahieren der Gasproben aus Löchern (1) in der Bodenoberfläche in der Nähe eines vermuteten Kabelfehlers (2) und das Ansaugen der Gasproben in eine Messkammer (13) aufweist, die wenigstens einen Festkörpergassensor (12) enthält,
- der dritte Schritt des Überwachens die Verwendung einer elektronischen Schaltung (22) aufweist, die mit den Festkörpergassensoren verbunden ist, um Signale zu erzeugen, welche die Reaktionen der Festkörpergassensoren darstellen, und
- der vierte Schritt des Bestimmens das Analysieren von Signalen zum Identifizieren des Vorhandenseins von für die unterirdischen Starkstromkabelfehler charakteristischen Gasen in den Gasproben aufweist.

15. Verfahren nach Anspruch 14, bei welchem die Gasproben in die Messkammer (13) unter Verwendung einer Sonde (4) gesaugt werden, die in die Löcher (1) in der Bodenfläche eingeführt wird.

16. Verfahren nach Anspruch 14 oder Anspruch 15, bei welchem
- eine Anzahl der Löcher (1) in die Bodenoberfläche auf einer Linie über einem unterirdischen Kabel (3) gebohrt wird,
- aus jedem der Löcher Gasproben entnommen werden und
- die höchste Konzentration von Gasen, die für einen unterirdischen Starkstromkabelfehler charakteristisch sind, zur Anzeige des Lochs genommen wird, das dem Kabelfehler (2) am nächsten liegt.

17. Verfahren nach einem der Ansprüche 13 bis 16, bei welchem die Festkörpergassensoren (12) Festkörpergassensoren mit unterschiedlichen Empfindlichkeitseigenschaften für verschiedene Gase sind.

18. Verfahren nach einem der Ansprüche 13 bis 17, bei welchem die Festkörpergassensoren (12) Metalloxidhalbleiter-Gassensoren sind.

19. Verfahren nach Anspruch 18, bei welchem die Metalloxidhalbleiter-Gassensoren (12) bei gewünschten Temperaturen mit Hilfe von Heizelementen betrieben werden, die in sie eingeschlossen sind.

20. Verfahren nach einem der Ansprüche 13 bis 19, bei welchem wenigstens vier Festkörpergassensoren (12) verwendet werden.

21. Verfahren nach einem der Ansprüche 14 bis 20, bei welchem eine Massendurchsatzsteuerung (20) zum Steuern des Durchsatzes der Gasproben in die Messkammer (13) verwendet werden.

22. Verfahren nach einem der Ansprüche 14 bis 21, bei welchem Eicheinrichtungen zum Überwachen der Signale, wenn reine Luft in die Messkammer (13) angesaugt wird, zur Bereitstellung einer Eichbezugsgröße zur Verwendung beim Analysieren der Signale eingesetzt werden.

23. Verfahren nach einem der Ansprüche 14 bis 22, bei welchem das Signal aus einem Sensor (15) für relative Feuchte, der in der Messkammer positioniert ist, dazu verwendet wird, die Reaktionen der Festkörpergassensoren (12) auf Änderungen der relativen Feuchte auszugleichen.

24. Verfahren nach einem der Ansprüche 14 bis 23, bei welchem das Signal aus einem Lufttemperatursensor (16) dafür verwendet wird, die Reaktionen der Festkörpergassensoren (12) auf Änderungen der Lufttemperatur auszugleichen.

25. Verfahren nach einem der Ansprüche 13 bis 24, bei welchem der Schritt des Bestimmens eine gewichtete Summe der Reaktionen der Festkörpergassensoren (12) verwendet.

26. Verfahren nach einem der Ansprüche 13 bis 24, bei welchem der Schritt des Bestimmens ein neurales Netzwerk verwendet, das zur Identifizierung von Proben der Gase aus den Reaktionen der Festkörpergassensoren (12) ausgebildet ist.

27. Verfahren nach einem der Ansprüche 13 bis 24, bei welchem der Schritt des Bestimmens eine Analyse aus der Liste einer Hauptkomponentenanalyse und einer Unterscheidungsfunktionsanalyse verwendet.

## Revendications

1. Détecteur de défauts électriques destiné à détecter les défauts électriques (2) dans les câbles électriques souterrains (3) comprenant :
une pluralité de capteurs de gaz à semi-conducteurs (12) installés dans une chambre de détection (13), une sonde creuse (4) connectée à la chambre de détection et conçue pour prélever le gaz à partir d'un trou (1) dans la surface de sol au-dessus d'un défaut électrique suspecté (2), des moyens pour aspirer l'échantillon de gaz à travers la sonde dans la chambre de détection, une circuiterie électronique (22) connectée aux détecteurs de gaz pour produire des signaux représentant les réponses des capteurs, des moyens (10) pour analyser lesdits signaux afin d'identifier la présence dans ledit échantillon de gaz de gaz caractéristiques des défauts du câble électrique souterrain, et des moyens (10) pour indiquer le résultat de l'analyse.

2. Appareil selon la revendication 1 dans lequel les capteurs de gaz à semi-conducteurs (12) sont des capteurs de gaz à semi-conducteurs avec des caractéristiques de sensibilité différentes aux différents gaz.

3. Appareil selon la revendication 1, dans lequel les capteurs de gaz à semi-conducteurs (12) sont des capteurs de gaz à semi-conducteurs d'oxyde de métal.

4. Appareil selon la revendication 3, dans lequel les capteurs de gaz à semi-conducteurs d'oxyde de métal (12) incorporent des éléments chauffants, et l'appareil comprend des moyens (21) pour alimenter en énergie et contrôler les températures desdits éléments chauffants et ainsi contrôler les températures de fonctionnement desdits capteurs de gaz à semi-conducteurs d'oxyde de métal.

5. Appareil selon l'une quelconque des revendications précédentes dans lequel il y a au moins quatre desdits capteurs de gaz à semi-conducteurs (12).

6. Appareil selon l'une quelconque des revendications précédentes comprenant un contrôleur de flux de masse (20) pour contrôler le flux dudit échantillon de gaz dans ladite chambre de détection (13).

7. Appareil selon l'une quelconque des revendications précédentes comprenant des moyens d'étalonnage conçus pour surveiller lesdits signaux lorsque l'air propre est aspiré dans ladite chambre de détection (13) et pour fournir une référence d'étalonnage pour utilisation dans l'analyse desdits signaux.

8. Appareil selon l'une quelconque des revendications précédentes comprenant un capteur d'humidité relative (15) positionné dans ladite chambre de détection (13) ainsi que des moyens connectés audit capteur d'humidité relative pour compenser les réponses des capteurs de gaz à semi-conducteurs (12) pour les changements d'humidité relative.

9. Appareil selon l'une quelconque des revendications précédentes comprenant un capteur de température d'air (16) ainsi que des moyens connectés audit capteur de température d'air pour compenser les réponses des capteurs de gaz à semi-conducteurs (12) pour les changements dans la température de l'air.

10. Appareil selon l'une quelconque des revendications 1 à 9 dans lequel les moyens d'analyse (10) sont agencés pour utiliser une somme pondérée des réponses desdits capteurs de gaz à semi-conducteurs (12).

11. Appareil selon l'une quelconque des revendications 1 à 9 dans lequel les moyens d'analyse (10) sont agencés pour utiliser un réseau neuronal entraîné pour identifier les échantillons desdits gaz à partir des réponses desdits capteurs de gaz à semi-conducteurs (12).

12. Appareil selon l'une quelconque des revendications 1 à 9 dans lequel les moyens d'analyse (10) sont agencés pour utiliser l'un des procédés de la liste d'analyse des composants principaux et discriminer l'analyse de fonction.

13. Procédé pour détecter des défauts électriques (2) dans des câbles souterrains (3) comprenant les étapes consistant à :
prélever des échantillons de gaz à partir des lieux proches d'un défaut électrique suspecté (2) d'un câble souterrain ;
exposer au moins un capteur de gaz à semi-conducteurs (12) audit échantillon de gaz ; et
surveiller les changements conséquents dans les réponses des capteurs de gaz à semi-conducteurs et
déterminer à partir de celles-ci une indication de la localisation du défaut électrique.

14. Procédé selon la revendication 13 dans lequel la première étape comprend l'extraction desdits échantillons de gaz à partir des trous (1) de la surface du sol proches dudit défaut du câble suspecté (2) et aspirer lesdits échantillons de gaz dans une chambre de détection (13) contenant au moins un capteur de gaz à semi-conducteurs (12), la troisième étape de surveillance comprend l'utilisation de circuiterie électronique (22) connectée auxdits capteurs de gaz à semi-conducteurs pour produire des signaux représentant les réponses desdits capteurs de gaz à semi-conducteurs, et la quatrième étape de détermination comprend l'analyse desdits signaux pour identifier la présence dans lesdits échantillons de gaz des gaz caractéristiques des défauts du câble électrique souterrain.

15. Procédé selon la revendication 14 dans lequel lesdits échantillons de gaz sont aspirés dans ladite chambre de détection (13) en utilisant une sonde (4) insérée dans lesdits trous (1) dans la surface du sol.

16. Procédé selon la revendication 14 ou 15 dans lequel plusieurs desdits trous (1) sont percés dans la surface du sol dans une ligne au-dessus d'un câble souterrain (3), lesdits échantillons de gaz sont prélevés de chacun des trous et, dans lequel la concentration la plus élevée des gaz caractéristiques d'un défaut du câble électrique souterrain est prise pour indiquer le trou le plus proche du défaut du câble (2).

17. Procédé selon l'une quelconque des revendications 13 à 16 dans lequel lesdits capteurs de gaz à semi-conducteurs (12) sont des capteurs de gaz à semi-conducteurs avec différentes caractéristiques de sensibilité aux différents gaz.

18. Procédé selon l'une quelconque des revendications 13 à 17 dans lequel les capteurs de gaz à semi-conducteurs (12) sont des capteurs de gaz à semi-conducteurs d'oxyde de métal.

19. Procédé selon la revendication 18 dans lequel lesdits capteurs de gaz à semi-conducteurs d'oxyde de métal (12) sont actionnés à des températures souhaitées au moyen d'éléments chauffants incorporés dans ceux-ci.

20. Procédé selon l'une quelconque des revendications 13 à 19 dans lequel au moins quatre capteurs de gaz à semi-conducteurs (12) sont utilisés.

21. Procédé selon l'une quelconque des revendications 14 à 20 dans lequel un contrôleur de flux de masse (20) est utilisé pour contrôler le flux desdits échantillons de gaz dans ladite chambre de détection (13).

22. Procédé selon l'une quelconque des revendications 14 à 21 dans lequel les moyens d'étalonnage conçus pour surveiller lesdits signaux lorsque l'air propre est aspiré dans la chambre de détection (13) sont utilisés pour fournir une référence d'étalonnage pour utilisation dans l'analyse desdits signaux.

23. Procédé selon l'une quelconque des revendications 14 à 22 dans lequel le signal d'un capteur d'humidité relative (15) positionné dans ladite chambre de détection est utilisé pour compenser les réponses des capteurs de gaz à semi-conducteurs (12) pour les changements dans l'humidité relative.

24. Procédé selon l'une quelconque des revendications 14 à 23 dans lequel le signal d'un capteur de température d'air (16) est utilisé pour compenser les réponses des capteurs de gaz à semi-conducteurs (12) pour les changements dans la température de l'air.

25. Procédé selon l'une quelconque des revendications 13 à 24 dans lequel l'étape de détermination utilise une somme pondérée des réponses desdits capteurs de gaz à semi-conducteurs (12).

26. Procédé selon l'une quelconque des revendications 13 à 24 dans lequel l'étape de détermination utilise un réseau neuronal entraîné pour identifier les échantillons desdits gaz à partir des réponses desdits capteurs de gaz à semi-conducteurs (12).

27. Procédé selon l'une quelconque des revendications 13 à 24 dans lequel l'étape de détermination utilise un de la liste d'analyse de composants principaux et discrimine l'analyse de fonction.
